# EUROPÄISCHE PATENTANMELDUNG

(11) **EP 1 123 686 A2**
(43) Veröffentlichungstag der Anmeldung: **16.08.2001**
(21) Anmeldenummer: 01101598.9
(22) Anmeldetag: 25.01.2001
(51) Int. Cl.: A61B 5/11

(54) **Verfahren und Vorrichtung zur Darstellung einer Bewegungsabweichung**

(30) Priorität: 09.02.2000 DE 10005955
(71) Anmelder: Werk, Roland, Dr. med. Dipl.-Biol., 97072 Würzburg (DE)
(72) Erfinder: Werk, Roland, Dr. med. Dipl.-Biol., 97072 Würzburg (DE)
(74) Vertreter: Tappe, Hartmut

(57) **Zusammenfassung**

Die Erfindung betrifft ein Verfahren und eine Vorrichtung zur Darstellung einer Bewegungsabweichung (20) gegenüber einer Referenzbewegung (17) durch Vergleich der ausgeführten Bewegung (11) mit der Referenzbewegung (17) mit den Verfahrensschritten:
- optische und/oder sensorische Erfassung der ausgeführten Bewegung (11) zur Digitalisierung der Bewegung;
- Überlagerung der digitalisierten ausgeführten Bewegung (11) mit der digitalisierten Referenzbewegung (17) zur Ermittlung einer Bewegungsabweichung (20);
- visuelle Darstellung der Referenzbewegung (17) und der Bewegungsabweichung (20) auf einer Displayeinrichtung (21).

## Beschreibung

Die vorliegende Erfindung betrifft ein Verfahren zur Darstellung einer Bewegungsabweichung gegenüber einer Referenzbewegung durch Vergleich der ausgeführten Bewegung mit der Referenzbewegung. Darüber hinaus betrifft die Erfindung eine Vorrichtung zur Darstellung einer Bewegungsabweichung gegenüber einer Referenzbewegung durch Vergleich der ausgeführten Bewegung mit der Referenzbewegung.

Zum Erlernen mehr oder weniger komplexer Bewegungsabläufe werden üblicherweise Bewegungstrainer eingesetzt, die die Bewegungen oder Bewegungsabläufe vorexerzieren. Nachfolgend versucht der Bewegungslernende, die Bewegung des Bewegungstrainers möglichst exakt, also mit möglichst geringen Abweichungen zu wiederholen. Diese Methode, die durch die menschliche Art des Erlernens von Bewegungen, nämlich durch Nachahmung, bestimmt ist, wird auf den unterschiedlichsten Gebieten praktiziert. Dabei kann es sich genauso um krankengymnastische, also Therapie-Bewegungen, wie Bewegungen zur Ausführung einer handwerklichen Tätigkeit oder auch Bewegungen zur Ausführung einer sportlichen Disziplin handeln. In jedem Fall setzt die für den Menschen typische Art des Erlernens von Bewegungen einen Bewegungstrainer voraus, der sowohl anleitet als auch korrigiert. Der durch die Notwendigkeit eines Bewegungstrainers bedingte Aufwand zum Erlernen von Bewegungen ist daher sowohl in finanzieller als auch zeitlicher Hinsicht beträchtlich. Zudem ist in der Regel der Besuch spezieller Trainingsstätten notwendig, an denen Bewegungstrainer ihre Dienste anbieten. Darüber hinaus sind, insbesondere in dem Fall, wenn Bewegungslernende in größeren Gruppen zusammengefasst sind, die Möglichkeiten, die individuell unterschiedlich bei den einzelnen Bewegungslernenden vorhandenen Voraussetzungen effektiv bei dem Erlernen der Bewegungen berücksichtigen zu können, begrenzt.

Darüber hinaus ist festzustellen, dass die unvermeidbaren individuellen Unterschiede zwischen den einzelnen Bewegungstrainern einer an sich wünschenswerten Standardisierung einer Lehr- oder Standardbewegung entgegenstehen. Daher erweist es sich in der Praxis als schwierig, in einer Mehrzahl jeweils verschiedenen Bewegungstrainern zugeordneter Gruppen einen einheitlich hohen Qualitätsstandard zu etablieren.

Der vorliegenden Erfindung liegt daher die Aufgabe zugrunde, ein Verfahren sowie eine Vorrichtung vorzuschlagen, das bzw. die ein für den Bewegungslernenden komfortableres und effektiveres Erlernen von Bewegungen ermöglicht.

Diese Aufgabe wird durch ein Verfahren mit den Merkmalen des Anspruchs 1 gelöst.

Hierzu weist das erfindungsgemäße Verfahren die Verfahrensschritte auf:
- optische und/oder sensorische Erfassung der ausgeführten Bewegung zur Digitalisierung der Bewegung;
- Überlagerung der digitalisierten ausgeführten Bewegung mit der digitalisierten Referenzbewegung zur Ermittlung einer Bewegungsabweichung;
- visuelle Darstellung der Referenzbewegung und der Bewegungsabweichung auf einer Displayeinrichtung.

Durch die optische und/oder sensorische Erfassung der ausgeführten Bewegung erfolgt eine Erfassung der ausgeführten Bewegung genau in der Art, wie sie von der Umgebung wahrgenommen wird, so dass eine objektive Darstellung der ausgeführten Bewegung in digitalisierter Form die Folge ist. Im Vergleich hierzu ist der Bewegungslernende bei der konventionellen Methode zur Kontrolle seiner Bewegung beschränkt auf subjektive Körperempfindungen oder auf das Urteil des Bewegungstrainers. Auch die Überlagerung der digitalisierten ausgeführten Bewegung mit der digitalisierten Referenzbewegung ist bei Anwendung der bekannten Lehrmethode mittels eines Bewegungstrainers nicht annähernd möglich. Insbesondere sind schon aufgrund des bei der bekannten Methode fehlenden übereinstimmenden Bezugssystems für die Bewegungen des Bewegungstrainers und die Bewegungen des Bewegungslernenden quantitative Abweichungen kaum definierbar. Zudem setzt ein Vergleich zwischen der Bewegung des Lernenden und der Bewegung des Trainers im bekannten System aufgrund der sich aus der Personendistanz ergebenden räumlichen Distanz der Bewegungsbezugssysteme die Fähigkeit beim Lernenden voraus, das Bezugssystem der eigenen Bewegung in das Bezugssystem der Bewegung des Trainers zu transformieren oder umgekehrt. In der Praxis führt dies beispielsweise dazu, dass Bewegungen des Trainers spiegelverkehrt wiederholt werden müssen, was für den einen oder anderen Bewegungslernenden bereits ein Problem darstellt, das die Bewältigung der eigentlichen Lernaufgabe, nämlich Korrektur oder Minimierung der Abweichungen zwischen den Bewegungen des Lernenden und den Bewegungen des Trainers, behindert. Genau diese Konzentration auf die wesentliche, zu lösende Aufgabe wird durch die visuelle Darstellung der Referenzbewegung und der Bewegungsabweichung auf einer Displayeinrichtung für den Bewegungslernenden ermöglicht, ohne dass der Bewegungslernende hierzu gedankliche Vorleistungen erbringen müsste.

Bei einer besonders vorteilhaften Variante des Verfahrens erfolgt parallel zur visuellen Darstellung der Referenzbewegung und der Bewegungsabweichung auf einer Displayeinrichtung eine Generierung von Aktorsignalen in Abhängigkeit von der Bewegungsabweichung und eine Beaufschlagung der wiederholt ausgeführten Bewegung mit den Aktorsignalen, um dem Bewegungslernenden neben der visuellen Darstellung eine beispielsweise durch mechanische Reize ausgelöste sensorische Unterstützung bei der Ausführung der korrekten Bewegung anbieten zu können.

Weiterhin ist auch eine Variante des Verfahrens denkbar, bei der die vorgenannte visuelle Darstellung der Referenzbewegung und der Bewegungsabweichung auf einer Displayeinrichtung nicht durch die Generierung von Aktorsignalen ergänzt, sondern vielmehr durch die Generierung von Aktorsignalen und Beaufschlagung der wiederholt ausgeführten Bewegung mit den Aktorsignalen ersetzt wird. Dies ist insbesondere bei Bewegungslernenden vorteilhaft, die nur über begrenzte sensorische Fähigkeiten verfügen, also beispielsweise nicht oder nur stark eingeschränkt sehfähig sind.

Eine Möglichkeit, die Aktorsignale dem sensorischen Erfahrungsschatz des Bewegungslernenden entsprechend zu gestalten, besteht darin, die Aktorsignale als mechanische Impulse auszubilden. Hierdurch ist es möglich, die Aktorsignale beispielsweise als Drucksignale auf die an einer Bewegung beteiligten Körperteile zu richten, um hierdurch eine Ausweichbewegung in der Richtung der gewünschten Bewegung des Körperteils zu implizieren und dadurch das Erlernen der Bewegung zu unterstützen.

Insbesondere im Hinblick auf das Erlernen von Reaktionsbewegungen, wie sie beispielsweise bei Partnerübungen in Zweikampfdisziplinen erforderlich sind, erweist sich eine Variante des Verfahrens als vorteilhaft, bei der die Referenzbewegung durch die zu einer Ausgangsbewegung antagonistische Bewegung (Antagoniebewegung) gebildet ist.

Hierbei kann die Ausgangsbewegung durch die digitalisierte Trainerbewegung als einheitliche Ausgangsbewegung standardisiert vorgegeben werden. Aus der Ausgangsbewegung kann nun rechnergestützt die ideale Antagoniebewegung erzeugt werden, die die Referenzbewegung für den Bewegungslernenden darstellt.

Als besonders vorteilhaft für die Simulation von Partnerübungen bei Kampfsportarten, wie beispielsweise den Kumite beim Karate, erweist es sich, wenn eine zusätzliche visuelle Darstellung der Ausgangsbewegung erfolgt.

Die erfindungsgemäße Vorrichtung, die zur Durchführung des vorstehend erläuterten Verfahrens besonders geeignet ist, umfasst die Merkmale des Anspruchs 6.

Erfindungsgemäß weist demnach die Vorrichtung auf:
- eine digitale optische Aufnahmeeinrichtung zur Bilderfassung der ausgeführten Bewegung,
- einen Prozessor zur Überlagerung der digitalisierten ausgeführten Bewegung mit der digitalisierten Referenzbewegung und zur Ermittlung der Bewegungsabweichung und
- eine Displayeinrichtung zur Darstellung der Bewegungsabweichung und der Referenzbewegung.

Bei einer vorteilhaften Ausführungsform der Vorrichtung ist ergänzend zur Displayeinrichtung ein Aktorsignalgenerator zur Generierung von Aktorsignalen in Abhängigkeit von der Bewegungsabweichung vorgesehen. Alternativ hierzu ist es auch möglich, eine Vorrichtung vorzusehen, die anstatt einer Displayeinrichtung zur Darstellung der Bewegungsabweichung und der Referenzbewegung eine Einrichtung zur Generierung von Aktorsignalen in Abhängigkeit von der Bewegungsabweichung aufweist.

Für den Fall, dass die Vorrichtung mit einer Displayeinrichtung versehen ist, erweist es sich als besonders vorteilhaft, wenn die Displayeinrichtung als eine Displaybrille ausgebildet ist, die einen Gebrauch der Vorrichtung ermöglicht, ohne dass sich hierdurch Bewegungsbeeinträchtigungen für den Bewegungslernenden ergeben.

Als besonders vorteilhaft für die Ausführung der Einrichtung zur Generierung von Aktorsignalen erweist es sich, wenn die Aktorsignale als Druckimpulse ausgebildet sind, die unmittelbar auf ein an der Ausführung der Bewegung beteiligtes Körperglied aufgebracht werden können.

Nachfolgend wird das Verfahren sowie eine zur Durchführung des Verfahrens besonders geeignete Vorrichtung unter Bezugnahme auf die Zeichnung näher erläutert. Es zeigen:
- **Fig. 1**: die Darstellung einer Verfahrensvariante mittels eines Blockschaltbilds;
- **Fig. 2**: eine mittels eines Blockschaltbild schematisch dargestellte Vorrichtung zur Durchführung des Verfahrens.

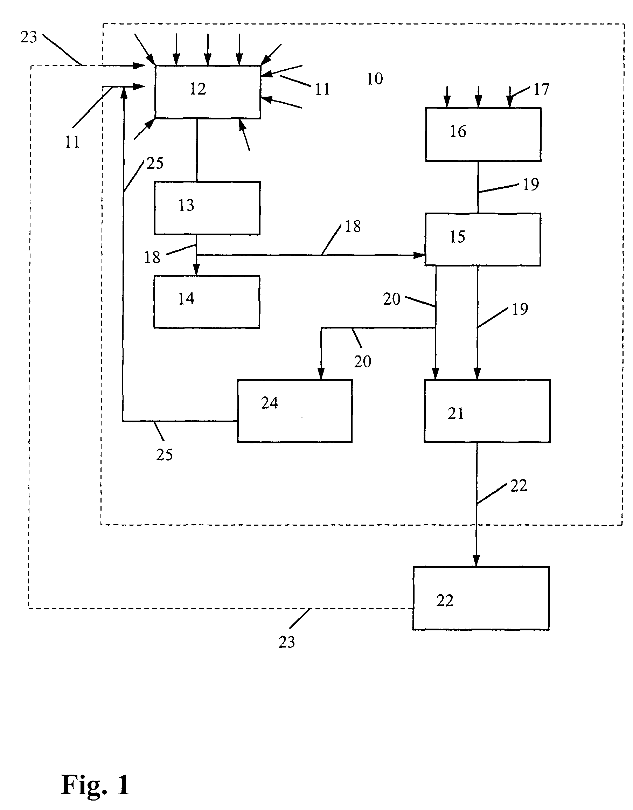

**Fig. 1** zeigt in einem Verfahrensschaubild eine Variante des Verfahrens, bei dem die in einem umgebenden Raum 10 ausgeführte, hier durch die Bewegungspfeile 11 angedeutete Bewegung von einer digitalen optischen und/oder allgemein sensorischen Aufnahmeeinrichtung 12 in ihrem zeitlichen Ablauf erfasst und zur Digitalisierung einer Bildverarbeitungseinrichtung 13 zugeführt wird. Eine von der Bildverarbeitungseinrichtung 13 digitalisierte Bildinformation 18 über die ausgeführte Bewegung 11 wird bei dem in **Fig. 1** dargestellten Ausführungsbeispiel sowohl einer digitalen Bildspeichereinrichtung 14 als auch einem Prozessor 15 zugeführt.

Der Prozessor 15 ist darüber hinaus mit einer Bildspeichereinrichtung 16 verbunden, die zur Speicherung einer Referenzbewegung 17 dient. Der Prozessor 15 ermöglicht eine Überlagerung der der ausgeführten Bewegung 11 zugeordneten Bildinformation 18 mit einer der Referenzbewegung 17 zugeordneten Bildinformation 19 zur Ermittlung einer Bewegungsabweichung 20 der ausgeführten Bewegung 11 gegenüber der Referenzbewegung 17. Sowohl die vom Prozessor 15 ermittelte Bewegungsabweichung 20 als auch die der Referenzbewegung 17 zugeordnete Bildinformation 19 werden auf einer Displayeinrichtung 21 dargestellt. Die hierdurch insgesamt auf der Displayeinrichtung 21 einem Bewegungslernenden zur Verfügung gestellte Bewegungsinformation 22 kann nun vom Bewegungslernenden 22 zur Beaufschlagung der von ihm ausgeführten Bewegung 11 mit entsprechenden Bewegungskorrekturen 23 genutzt werden, mit der Folge, dass die bei einer wiederholt ausgeführten Bewegung 11 gegenüber der Referenzbewegung 17 durch den Prozessor 15 ermittelte Bewegungsabweichung 20 verringert oder gar eliminiert werden kann.

Zur Unterstützung der Reaktion des Bewegungslernenden 22 zur Verringerung bzw. Eliminierung der Bewegungsabweichung 20 der ausgeführten Bewegung 11 gegenüber der Referenzbewegung 17 kann die Bewegungsabweichung 20 auch als Eingangsgröße für eine Einrichtung 24 zur Generierung von Aktorsignalen 25 genutzt werden. Die Aktorsignale 25 dienen zur Beaufschlagung der Bewegung 11 mit dem Ziel, die vom Prozessor 15 ermittelte Bewegungsabweichung 20 der ausgeführten Bewegung 11 gegenüber der Referenzbewegung 17 weiter zu reduzieren bzw. zu eliminieren.

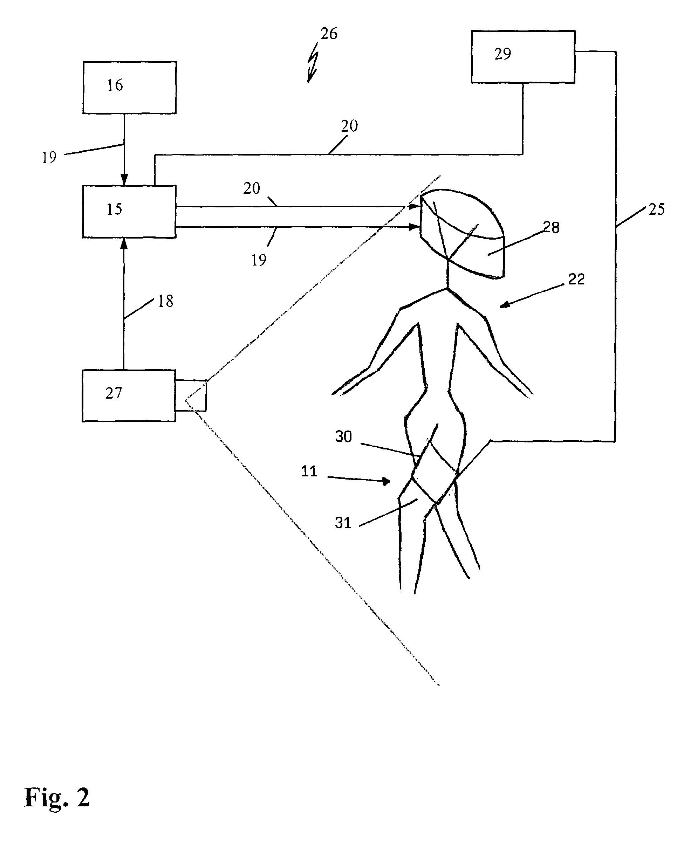

**Fig. 2** zeigt eine mögliche Ausführungsform einer Vorrichtung 26 zur Durchführung des anhand **Fig. 1** erläuterten Verfahrens. Die Vorrichtung 26 umfasst in dem in **Fig. 2** dargestellten Fall eine CCD-Kamera 27, die einerseits zur optischen Erfassung der von einem Bewegungslernenden 22 ausgeführten Bewegung 11 dient, andererseits auch die Digitalisierung der optisch erfassten Bewegung 11 ermöglicht, derart, dass am Ausgang der CCD-Kamera 27 die ausgeführte Bewegung 11 als digitalisierte Bildinformation 18 zur Verfügung steht. Neben der CCD-Kamera 27 umfasst die Vorrichtung 26 den Prozessor 15, der an die Bildspeichereinrichtung 16 angeschlossen ist und von dieser mit digitalisierter Bildinformation 19 betreffend eine digital abgespeicherte Referenzbewegung 17 **(Fig. 1)**versorgt wird. Als weitere Einrichtung umfasst die Vorrichtung 26 eine Displaybrille 28 sowie einen Aktorsignalgenerator 29, der entsprechend der vom Prozessor 15 ermittelten Abweichung 20 zwischen der ausgeführten Bewegung 11 und der Referenzbewegung 17 Aktorsignale 25 generiert, die über einen, hier in Form einer Manschette, ausgebildeten Aktorsignalgeber 30 auf einen Oberschenkel 31 des Bewegungslernenden 22 wirken. Derart kann beispielsweise durch einen an der Unterseite des Oberschenkels 31 vom Aktorsignalgeber 30 erzeugten Druckimpuls ein Anheben des Oberschenkels 31 zur Beeinflussung der ausgeführten Bewegung 11 induziert werden.

Die Darstellung der Bewegungsinformationen 19, 20 als Displayinformation auf der Displaybrille 28 ermöglicht es dem Bewegungslernenden 22, sich unabhängig von weiteren visuellen Umgebungseinflüssen auf die Ausführung von Bewegungskorrekturen zu konzentrieren, die eine Reduzierung bzw. Eliminierung der Bewegungsabweichung 20 gegenüber der Referenzbewegung 17 unterstützen.

## Patentansprüche

1. Verfahren zur Darstellung einer Bewegungsabweichung gegenüber einer Referenzbewegung durch Vergleich der ausgeführten Bewegung mit der Referenzbewegung
**gekennzeichnet** durch
die Verfahrensschritte:
- optische und/oder sensorische Erfassung der ausgeführten Bewegung (11) zur Digitalisierung der Bewegung;
- Überlagerung der digitalisierten ausgeführten Bewegung (11) mit der digitalisierten Referenzbewegung (17) zur Ermittlung einer Bewegungsabweichung (20);
- visuelle Darstellung der Referenzbewegung (17) und der Bewegungsabweichung (20) auf einer Displayeinrichtung (21).

2. Verfahren nach Anspruch 1,
**gekennzeichnet** durch
die Generierung von Aktorsignalen (25) in Abhängigkeit von der Bewegungsabweichung (20) und Beaufschlagung der wiederholt ausgeführten Bewegung (11) mit den Aktorsignalen.

3. Verfahren nach Anspruch 2 oder 3,
dadurch **gekennzeichnet,**
dass die Aktorsignale (25) als mechanische Impulse ausgebildet sind.

4. Verfahren nach einem oder mehreren der vorangehenden Ansprüche,
dadurch **gekennzeichnet,**
dass die Referenzbewegung durch die zu einer Ausgangsbewegung antagonistische Bewegung (Antagoniebewegung) gebildet ist.

5. Verfahren nach Anspruch 4,
**gekennzeichnet** durch
eine zusätzliche visuelle Darstellung der Ausgangsbewegung.

6. Vorrichtung zur Darstellung einer Bewegungsabweichung gegenüber einer Referenzbewegung durch Vergleich der ausgeführten Bewegung mit der Referenzbewegung,
**gekennzeichnet** durch:
- eine digitale optische Aufnahmeeinrichtung (27) zur Bilderfassung der ausgeführten Bewegung (11);
- einen Prozessor (15) zur Überlagerung der digitalisierten ausgeführten Bewegung (11) mit der digitalisierten Referenzbewegung (17) und zur Ermittlung der Bewegungsabweichung (20);
- eine Displayeinrichtung (21) zur Darstellung der Bewegungsabweichung (20) und der Referenzbewegung (17).

7. Vorrichtung nach Anspruch 6,
**gekennzeichnet** durch
einen Aktorsignalgenerator (29) zur Generierung von Aktorsignalen (25) in Abhängigkeit von der Bewegungsabweichung (20).

8. Vorrichtung nach Anspruch 6 oder 7,
dadurch **gekennzeichnet,**
dass die Displayeinrichtung als eine Displaybrille (28) ausgebildet ist.

9. Vorrichtung nach Anspruch 7 oder 8,
dadurch **gekennzeichnet,**
dass die Aktorsignale (25) als Druckimpulse ausgebildet sind, die über einen Aktorsignalgeber (30) unmittelbar auf ein an der ausführenden Bewegung (11) beteiligtes Körperglied (31) wirken.
